# EUROPEAN PATENT SPECIFICATION

(11) **EP 3 423 500 B1**
(45) Date of publication and mention of the grant of the patent: **07.06.2023**
(21) Application number: 16892078.3
(22) Date of filing: 03.03.2016
(51) Int. Cl.: C08F 22/06, C08F 8/44, C08F 8/12, A61K 6/887, A61L 15/24, C08L 29/10, C08L 31/06, C08L 33/02, C08F 222/02, A61K 6/17, A61K 6/35, A61K 6/889, C08L 1/28, C08L 5/04

(54) **FINE AND UNIFORM METHYL VINYL ETHER-MALEIC ACID SALT COPOLYMERS AND THEIR USE IN ORAL CARE AND PHARMACEUTICAL APPLICATIONS**
FEINE UND EINHEITLICHE METHYLVINYLETHER-MALEINSÄURE-SALZ-COPOLYMERE UND DEREN VERWENDUNG IN DER ORALEN PFLEGE UND IN DER PHARMAZIE
COPOLYMÈRES FINS ET UNIFORMES DE SELS D'ÉTHER VINYLIQUE DE MÉTHYLE-ACIDE MALÉIQUE, ET LEUR UTILISATION DANS LES APPLICATIONS DE SOINS BUCCAUX ET PHARMACEUTIQUES

(43) Date of publication of application: 09.01.2019
(73) Proprietor: Boai NKY Medical Holdings Ltd., 454450 Jiaozuo, Henan (CN)
(72) Inventor: ULMER, Herbert Wilhelm, Jiaozuo Henan 454450 (CN); WANG, Jianqiang, Jiaozuo Henan 454450 (CN); WANG, Long, Jiaozuo Henan 454450 (CN); YUAN, Guodong, Jiaozuo Henan 454450 (CN); SUN, Jianxin, Jiaozuo Henan 454450 (CN); SUN, Xudong, Jiaozuo Henan 454450 (CN)
(74) Representative: V.O.
(86) International application number: PCT/CN2016/075552
(87) International publication number: WO 2017/147887

(56) References cited:
- WO-A1-02/48213
- CN-A- 105 147 527
- US-A- 5 304 616
- US-A- 5 525 652

## Description

The invention relates to fine and uniform copolymer powders of partial mixed metal salts of lower alkyl vinyl ether-maleic acid copolymers having a defined particle size distribution. In particular, it relates to powders that are suitably used in the areas of oral care and pharmaceuticals and to methods for preparing the powders.

The inorganic salt derivatives of methyl vinyl ether/maleic acid copolymers have significant use applications in the area of bioadhesives and mucosal adhesives in various oral care and pharmaceutical applications. The use of such polymers as denture adhesives is especially well documented.

US Patent No. 4,980,391 issued to Kumar et al. and assigned to the Warner-Lambert Company, discloses denture formulations consisting of partial mixed calcium and sodium salts of methyl vinyl ether-maleic acid copolymers and sodium carboxymethylcellulose in a petrolatum/mineral oil cream base.

US Patent No. 5,298,534 issued to Prosise et al. and assigned to ISP Investments Inc., discloses denture formulations consisting of partial mixed metal salts of lower alkyl vinyl ether-maleic acid copolymers and crosslinked non-ionic natural guar gum in oil base formulations.

US Patent No. 5,525,652 issued to Clarke et al. and assigned to the Block Drug Company, Inc., discloses denture adhesive preparations that utilize partially neutralized mixed sodium, magnesium and zinc salts of alkyl vinyl ether-maleic acid copolymers and sodium carboxymethylcellulose as the main adhesive components.

US Patent Nos. 5,304,616 and 6,617,374 issued to Rajaiah et al. and assigned to The Procter & Gamble Company, discloses optimized partial mixed salts of lower alkyl vinyl ether-maleic acid copolymers containing the cationic salt functions: strontium, zinc, iron and/or calcium cations and denture preparations there of.

The above patents all look to improve on problems long associated with denture adhesives, which include: phase separation of the formulation, lack of adhesion strength, lack of adhesion durability and oozing of the denture adhesive composition from under the denture plate.

US Patent No. 5,304,616 indicates in the text that the partial mixed salts of lower alkyl vinyl ether-maleic acid copolymers should be passed through a 140 to 200 mesh sieve before using. US Patent No. 5,525,652 indicates in the text that the mixed salts should be passed through a 100-mesh sieve prior to formulating. Mesh sizes of 100 and 200 corresponds to a micron size of 149 and 75, respectively. However, there is no reference to why this particular mesh size is needed and/or the effect of the mesh size on denture performance. Rather, it is expected that the passing reference of sieving is mainly conducted to remove oversize particles.

US Patent Appl. No. 2002/0111394 relates to denture adhesive powder compositions having improved adhesion properties. It generally teaches that an average particle size of between about 5 and about 200 microns diameter is acceptable for the partial mixed salt of lower alkyl vinyl ether-maleic acid copolymer. The specific examples utilize a polymer particles size of about 100 microns average diameter. US Patent Appl. No. 2002/0111394 is silent about particle uniformity and its effect on finished formulation properties fails to provide a teaching to arrive at the specific particle size and its uniformity as disclosed herein. In fact, the patent is focused on testing polymers of about 100 microns in average size to represent expected polymer performance.

Quite unexpectedly, the present inventors discovered that the overall size and uniformity of the resultant powders of lower alkyl vinyl ether-maleic anhydride partial mixed salts have a profound effect on the polymer's performance in applications in which the powder is used "as is" to formulate the finished denture adhesive formulation. Similar benefits can also be observed for other powder applications, such as in wound care.

The present invention pertains to a composition of powders according to claim 1. Claims 2-13 disclose further embodiments.

In one embodiment, the invention provides a composition of powders of partial mixed metal salts of C₁-C₄ alkyl vinyl ether-maleic acid copolymers having:
(i) a median particle size by volume (Dv50) of less than or equal to 25 microns; a maximum particle diameter below which 90% of the sample volume exists (Dv90) of less than or equal to 50 microns; and
(ii) a particle uniformity reflected by a ratio between the maximum particle diameter below which 90% of the sample volume exists and the maximum particle diameter below which 10% of the sample volume exists (Dv90/Dv10 ratio) of less than or equal to 10, preferably wherein the Dv90/Dv10 ratio is less than or equal to 6, wherein Dv10, Dv50 and Dv90 are measured according to the example in the description,
and wherein from about 10 to about 90 mol % of the carboxylic units in the polymer are converted to a mixture of metal salts.
A composition of powders of partial mixed metal salts of lower alkyl vinyl ether-maleic acid copolymers according to the invention is not disclosed or suggested in the art.

US 5,304,616 relates to particles of the same type of polymer, and to its application as denture adhesive composition. However, it fails to disclose any defined mean particles size nor a size uniformity, let alone that it suggests these parameters to be of relevance for improving adhesion properties. Instead, US 5,304,616 teaches to modify the type of cations in the mixed partial salts, more particularly to use a combination of strontium and zinc cations.

CN105147527 discloses denture adhesives based on ultrafine powders (Dv50 ≤ 25 microns and Dv90 ≤ 30 microns) of methyl vinyl ether/maleic acid inorganic salt copolymers. The focus of CN105147527 is solely on the fine particle size and in no way it teaches or suggests that a particle uniformity reflected by a Dv90/Dv10 ratio ≤ 10 is a major determinant for the adhesion performance of a powdered composition nor does it suggest tuning the method of manufacturing for making uniform particles.

Both the size of the powder particles (Dv50 and Dv90) and its uniformity (Dv90/Dv10 ratio) need to be tightly controlled in order to obtain optimum performance for applications in which the powder is used "as is" in the formulation. In one embodiment, the Dv90/Dv10 ratio is less than or equal to 8, preferably less than or equal to 7, more preferably the Dv90/Dv10 ratio is less than or equal to 6, like ≤ 5.8, <_ 5.5, ≤5.1, or ≤ 5. In a specific aspect, Dv90/Dv10 ratio is less than or equal to 4, like ≤ 3.8, <_ 3.6, ≤3.4 or ≤ 3.2.

In a specific aspect, the composition of powders has a Dv50 of less than 15 microns, a Dv90 of less than 25 microns and a Dv90/Dv10 ratio of less than 6.

It is preferred that the oversize fraction equal to or less than 1% by volume is not greater than 75 microns, preferably not greater than 50 microns. The main reason for this criterion is to remove the possibility for any negative aesthetic feel attributes. Larger particles can give the formulation a "grittiness" reduces its creamy smooth feel qualities. The larger particles also give the resultant formulation a "dull" appearance rather than the usual glossy sheen.

A composition of powders of partial mixed metal salts as provided herein preferably comprises lower alkyl vinyl ether-maleic acid copolymers of the general Formula I 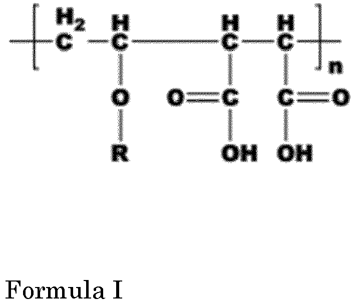 wherein R is C₁ to C₄ alkyl group. Very good results are obtained if the alkyl vinyl ether is methyl vinyl ether.

From about 10 to about 90 mol % of the carboxylic units in the vinyl ether-maleic acid polymer are converted to a mixture of metal salts. The metal salt may be selected from the group consisting of sodium, calcium, strontium, zinc, magnesium, iron and potassium. Fine powders of methyl vinyl ether-maleic acid mixed calcium/sodium salt copolymers and/or methyl vinyl ether-maleic acid mixed calcium/zinc salt copolymers are especially preferred. In one embodiment, the partial mixed metal salts are sodium and calcium salts. In another embodiment, the partial mixed metal salts are calcium and zinc salts.

Accordingly, in one embodiment the invention provides a composition of powders of partial mixed metal salts of lower alkyl vinyl ether-maleic acid copolymers of the general Formula I, wherein R is C₁ to C₄ alkyl group, and the carboxylic acid functionality being a mixture of free acid and 10-90 mole % cationic salt function selected from the group: sodium, calcium, zinc, strontium, magnesium, potassium and iron, having:
(i) a median particle size by volume (Dv50) of less than or equal to 25 microns; a maximum particle diameter below which 90% of the sample volume exists (Dv90) of less than or equal to 50 microns; and
(ii) a particle uniformity reflected by a ratio between the maximum particle diameter below which 90% of the sample volume exists and the maximum particle diameter below which 10% of the sample volume exists (Dv90/Dv10 ratio) of less than or equal to 10, preferably wherein the Dv90/Dv10 ratio is less than or equal to 6.

The fine polymer powders of the invention are especially suited for enhancing the product properties in which the polymer powder is used "as is" in the finished formulation. These applications include cream and powder based denture adhesives, denture wafers or film based denture adhesives and wound dressings where the alkyl vinyl ether-maleic acid mixed partial salt powders are used as the primary or secondary ingredient in said formulations.

Denture adhesive compositions are generally prepared from natural or synthetic polymeric materials suspended in an oil-base carrier or used directly as a powder. Upon contact with water (i.e. saliva) the polymeric materials have the property of swelling and form a gelatinous mass that can fill the space, act as a cushion and provide adhesion to hold the denture plate to the gum tissue.

The alkyl vinyl ether-maleic acid partial metal salt powders are directly formulated in the denture adhesive cream or powder and represent a critical adhesive constituent in denture formulations. The rate and uniformity of how the polymeric adhesive hydrates to give the final adhesive gelatinous mass is imperative to the final adhesive properties. It is this critical step of hydration and wet adhesion that is especially affected by the polymer particle size used to make the initial formulation.

It has been discovered that formulations using the fine and uniform polymer powders identified in this invention result in the quick and uniform formation of continuous adhesive films upon wetting. These films have less "defects" in the resultant continuous structure, which enhances strength and durability, both short and long term. In one embodiment, the copolymer in a composition of the invention has a 5% aqueous solution viscosity of greater than 60 cps, preferably greater than 80 cps and most preferably greater than 100 cps. In addition, the fine powders formulate to give more homogeneous formulations that enhance their stability and aesthetic properties. For example, cream denture formulations using powders of this invention have a more smooth and creamy consistency that is especially pleasing to the end-user.

A composition of powder as provided herein has a number of advantageous uses. For example, provided is a personal care, oral care, health care or pharmaceutical product comprising the composition of powders. Preferably, the oral care product is a denture adhesive. In a specific aspect, the invention provides a denture adhesive comprising partial calcium and sodium mixed salt of methyl vinyl ether-maleic acid copolymer. In another preferred embodiment, the partial calcium and zinc mixed salt of methyl vinyl ether-maleic acid copolymer are used. A denture adhesive may contain 10-60 weight percent, like 20-50 weight%, of the calcium and sodium mixed salt of methyl vinyl ether-maleic acid copolymer.

Pharmaceutical applications include adhesive powder coatings and wound applications where the copolymer powders are used directly, "as is", when producing the wound-covering or mucosal adhesive product. Such is the application as identified in US Patent No. 4,782,642 where occlusive wound dressings are prepared from ingredients that include the partial mixed calcium/sodium salt of methyl vinyl ether-co-maleic acid for treatment of skin lesions that emit large amounts of fluids. The fine powders of this invention are suitably used to enhance the uptake of wound fluid and develop a more continuous and durable wound covering upon hydration. Hence, exemplary health care products include a wound dressing, or a mucosal adhesive or bioadhesive.

The homogeneous copolymers of the invention are obtainable by first copolymerizing the C₁-C₄ alkyl vinyl ether and maleic anhydride monomers in a suitable solvent system to give the alkyl vinyl ether-co-maleic anhydride copolymer. In one embodiment, the invention provides a method of producing a composition of powders of the invention comprising of the steps: (i) copolymerizing the C₁-C₄ alkyl vinyl ether with maleic anhydride in a suitable solvent; (ii) hydrolyzing the resultant maleic anhydride copolymer and reacting with metal cations either in the form of a base or a salt or an oxide in an aqueous medium to give the partial mixed metal salt; (iii) drying the mixed metal salt copolymer and (iv) milling the mixed metal salt copolymer to the particle limits described herein above.

Any solvent system can be used that results in the efficient copolymerization of the monomers to high molecular weight copolymers. Suitable solvents include hydrocarbons, aromatics, esters and ethers. For instance, heptane, cyclohexane, benzene, ethyl acetate, isopropyl acetate, methyl vinyl ether, or any mixture thereof, is used.

The polymerization reaction can be initiated by means known in the art. Preferred free radical initiators are either organic peroxides or azo initiators such as: di-t-butyl peroxide, lauroyl peroxide, decanoyl peroxide, t-butyl peroxypivalate, 2,2'-azobis(isobutyronitrile) and 2,2'-azobis(2-methylbutyronitrile) and dimethyl 2,2'-azobis(2-methylpropionate), although other initiators known in the art may be used as well.

The polymerization reaction is carried out at a suitable temperature, generally in the range of about 50°-150° C, preferably 60°-100° C. The exact temperature reaction temperature is generally decided by the decomposition rate of the initiator system being used. Generally reaction temperatures are employed in which the initiator's decomposition half-life is between about 15 minutes and about 5 hours, more preferably between about 1 hour and about 3 hours. Either single initiator or multi initiator systems can be employed in the reaction. The monomer(s) can be added all up-front into the polymerization reactor or can be fed continuously or batch into the reactor over a predetermined time period. The polymerization process can be conducted either in batch or continuous mode. Copolymers of methyl vinyl ether-co-maleic anhydride (PVM/MA) having molecular weights in the range of about 500,000 and 3,000,000 g/mol are preferred.

The resultant alkyl vinyl ether-co-maleic anhydride copolymer is then hydrolyzed and reacted with the desired cationic metal to give the partial copolymer mixed salt. The reaction is carried out so that about 10 to about 90 mole % of the carboxylic units are converted to a mixture of metal salts. The cationic metals of this invention include: sodium, calcium, strontium, zinc, magnesium, iron and potassium and mixtures thereof. Preferred mixed polymer salt combinations are: sodium/calcium and calcium/zinc. Partial copolymer mixed salts can be prepared by the reaction of the alkyl vinyl ether-co-maleic anhydride/acid copolymer with desired metal cations either in the form of a base or a salt or an oxide in an aqueous medium.

The partial mixed metal salt of lower alkyl vinyl ether-maleic acid copolymers are dried and then milled to the powder specifications outlined in this invention. It has been discovered that jet-milling is especially suited for making the powders to the desired particle size specifications of the invention.

The copolymer may be mixed with at least one co-ingredient prior to drying or milling and then milled together to give the final powder product. Suitable co-ingredients include one or more of the following: carboxymethylcellulose and its sodium salt, polyvinylpyrrolidone, alginates, polyacrylic acid based copolymers, polymethacrylate based copolymers, poly(ethylene oxide), silicon dioxide, natural gums, cellulose derivatives, saccharide derivatives, synthetic polymers and mixtures thereof.

A further aspect relates to a composition or formulation comprising a copolymer powder of the invention. For example, the invention provides a personal care, oral care, health care or pharmaceutical product comprising the product obtained by the method described herein above.

Due to the unique and enhanced properties of the fine powders, the copolymer powders of the invention find application in various applications that directly utilize partial mixed metal salts of lower alkyl vinyl ether-maleic acid copolymers "as is". These applications include: oral care - denture adhesives, adhesive powder coatings and buccal patches and pharmaceuticals - wound coatings, mucosal adhesives and bioadhesives.

In addition to enhancing the mechanical testing properties of the finished formulation, incorporation of the copolymer powders can also have a drastic effect at improving the aesthetic properties of the finished formulation. Improved product texture, mouth feel and stability are enhanced.

All of the compositions, methods and experiments disclosed and claimed herein can be made and executed without undue experimentation in light of the present invention. While the compositions, methods and experiments of this invention have been described in terms of preferred embodiments, it will be apparent to those skilled in the art that variations may be applied to the compositions and methods and in the steps or in the sequence of steps of the method described herein without departing from the concept, spirit and scope of the invention. All modifications and applications apparent to those skilled in the art are deemed to be within the spirit, scope and concept of the invention as defined in the appended claims.

### LEGEND TO THE FIGURES

Figure 1: Tensile tests run at 37° C for various PVM/MA - Ca/Na partial mixed salts having varying particle sizes. For details, see example 1.
Figure 2: Particle size plot for PVM/MA-Ca/Na partial mixed salt of invention. For details, see example 2.
Figure 3: Particle size plot for Gantrez^{®} MS-955 (commercial product). For details, see example 2.
Figure 4: Adhesive testing comparison at 37° C of PVM/MA-Ca/Na of the invention and Gantrez^{®} MS-955. For details, see example 2.
Figure 5: Comparison of PVM/MA-Ca/Na of the invention and Gantrez^{®} MS-955 adhesion values at varying hydration times at 37° C. For details, see example 2.
Figure 6: Transmittance Spectra FTIR comparison of PVM/MA-Ca/Na Invention and Gantrez^{®} MS-955. For details, see example 2.

### EXPERIMENTAL SECTION

### Example 1: The effect of particle size on hydration rates and adhesion values

A batch of methyl vinyl ether-maleic acid partial calcium/sodium mixed salt (PVM/MA-Ca/Na) was prepared using polymerization techniques well known in the art. The resultant viscous solution was dried and the powder milled in a conventional impact mill. The resultant fine powder was then divided into five particle size regimes by sieving techniques. The following table 1 gives an overview of the resultant particle size fractions.

**Table 1**

| | |
|---|---|
| Particle size PVM/MA-Ca/Na fraction 1 | < 15 microns |
| Particle size PVM/MA-Ca/Na fraction 2 | 15 - 25 microns |
| Particle size PVM/MA-Ca/Na fraction 3 | 25 - 55 microns |
| Particle size PVM/MA-Ca/Na fraction 4 | 55 - 105 microns |
| Particle size PVM/MA-Ca/Na fraction 5 | 105 - 300 microns |

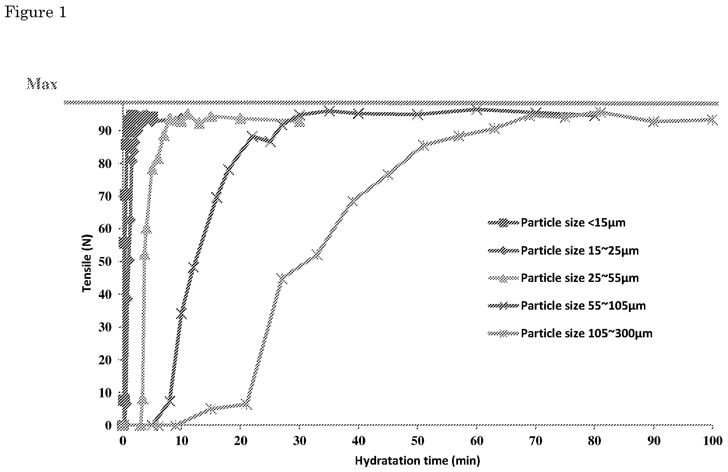

One gram of each of the above PVM/MA-Ca/Na fractions was quickly dispersed in 4 g distilled water and placed between two polymethylmethacrylate testing plates and placed in a tensile test machine at 37° C. The testing conditions were used to mimic the oral cavity. The resultant adhesion tests for the various powder sizes are summarized in Figure 1.

As shown in Figure 1, the particle size of the PVM/MA-Ca/Na copolymer has a drastic effect on the hydration rate of the particles and the formation of the resultant continuous structure. What is interesting to note is that the uniformity of the adhesion buildup also seems to be improved as compared to the larger particle size curves (i.e. the curves are more smooth). It should also be pointed out that the maximum adhesion level of about 116 N is the same for all particles size fractions.

The following table 2 summarizes the time needed to reach maximum adhesion for the various sized PVM/MA-Ca/Na particles.

**Table 2**

| PVM/MA-Ca/Na particle size | Hydratation time | Hydratation rate |
|---|---|---|
| (µm) | (min) | (N/min) |
| <15 | 1 | 116.34 |
| 15∼25 | 2.75 | 59.93 |
| 25∼55 | 8 | 22.36 |
| 55∼105 | 30 | 5.54 |
| 105∼300 | 69 | 2.41 |

It is clear that hydratation of particles of about 50 microns or less occurs quickly and uniformly. Though the higher particle size materials eventually reach a similar adhesion maximum of about 116 N, the process takes much longer. In the case of denture adhesives, this is undesirable because the denture can dislodge with chewing, negatively effecting the adhesives strength and long-term durability.

In addition, denture adhesive formulations are usually a mixture of adhesive polymers. It is essential that the various polymer ingredients hydrate at similar rates in order that the hydrated gelatinous mass forms uniformly to obtain maximum adhesion properties. Adhesive components that hydrate at vastly different rates result in "defects" in the formation of the continuous gelatinous mass/film. These "defects" act as weak points in the hydrating structure resulting in inferior adhesion properties, which limits the maximum adhesion obtained and adhesion durability of the formulation.

### Example 2: Comparison studies of invention and existing commercial product

A large-scale batch of PVM/MA-Ca/Na copolymer was produced, dried and jet-milled to give the fine particle size dimensions outlined in this invention. This material was compared to the commercial PVM/MA-Ca/Na copolymer, Gantrez^{®} MS-955 manufactured by Ashland Inc. and used as a base ingredient in many denture adhesive formulations.

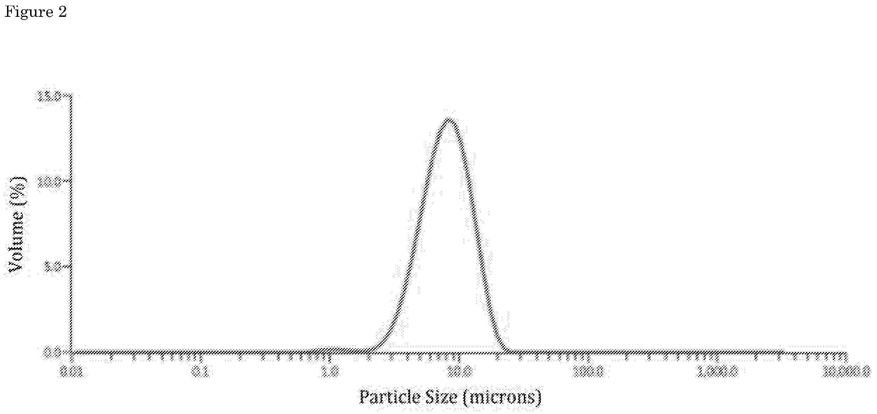

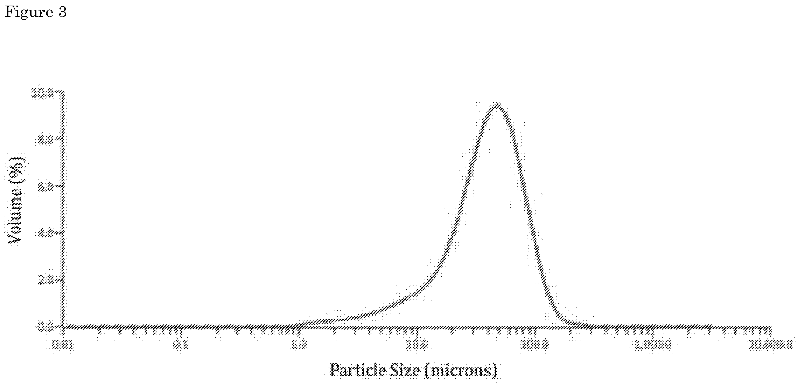

Particle size measurements were conducted on both powders using a Malvern Mastersizer 3000 particle size analyzer. The resultant particle size plots for the PVM/MA-Ca/Na of the invention and Gantrez^{®} MS-955 are shown in Figures 2 and 3, respectively. The following table 3 is a summary of the particle size values for the two polymer materials.

**Table 3**

| Polymer | Dv10¹ (µm) | Dv50² (µm) | Dv90³ (µm) | Dv90/Dv10 ratio | Maximum particle size (µm) |
|---|---|---|---|---|---|
| PVM/MA-Ca/Na invention | 4.4 | 8.0 | 13.5 | 3.1 | ≈ 25 |
| Gantrez^{®} MS-955 | 12.0 | 41.1 | 86.3 | 7.2 | ≈ 250 |

| | | | | | |
|---|---|---|---|---|---|
| 1) The maximum particle size diameter below which 10% of the sample volume exists 2) The maximum particle size diameter below which 50% of the sample volume exists also called median particle size by volume 3) The maximum particle size diameter below which 90% of the sample volume exists | | | | | |

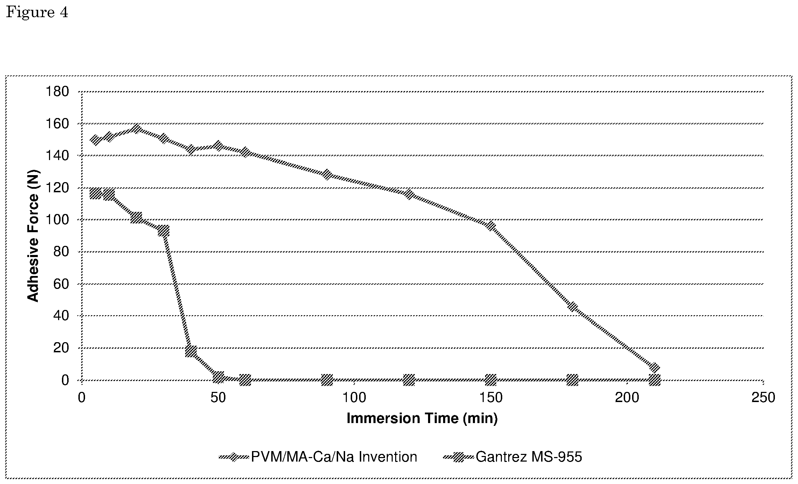

The two powders were formulated as 20% solids in purified water and allowed to completely hydrate. Once hydrated, 4 g of each hydrated mass was placed between two polymethylmethacrylate testing plates and placed in a tensile test machine and submerged in artificial saliva at 37° C. The adhesion test was then conducted at various time periods mimicking the oral cavity and the effect of chewing. The results are shown in Figure 4.

As can be seen from the graph, the adhesion profile for the PVM/MA-Ca/Na invention product shows significantly improved maximum adhesion and longevity of adhesion as compared to the commercial Gantrez^{®} MS-955 product. The ultra-fine and uniform particle distribution of the invention show a vastly superior adhesion profile that is especially desirable for denture adhesive applications.

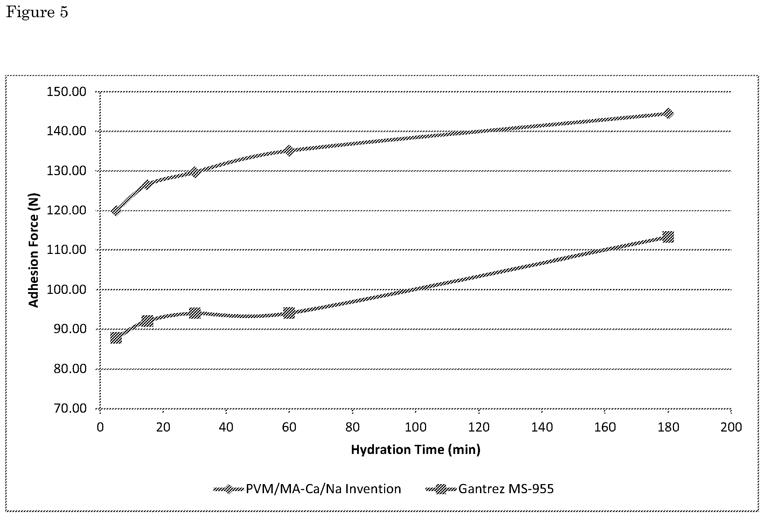

The two products were then compared by a second adhesion test conducted in the same manner as earlier, but the adhesion measurements were taken at various hydration times. These results are shown in Figure 5.

As can be seen from Figure 5, the fine particle PVM/MA-Ca/Na invention shows significantly improved adhesion values at short hydration times as compared to the commercial Gantrez^{®} MS-955. The fine powder of the invention hydrates readily and uniformly generating a continuous adhesive matrix that quickly generates the desired adhesion values. This is especially important for applications in which the powder should generate an adhesive matrix within a short time period such as is the case with denture adhesives.

Table 4 summarizes various particles size details for multiple batches of PVM/MA-Ca/Na of the invention. Batches 1-4 of the PVM/MA-Ca/Na materials possessed similar adhesion profiles to batch 1, which was the original material of the invention identified in Figure 4.

**Table 4**

| PVM/MA-Ca/Na invention | Dv10 (µm) | Dv50 (µm) | Dv90 (µm) | Dv90/Dv10 ratio |
|---|---|---|---|---|
| Batch 1 | 4.4 | 8.0 | 13.5 | 3.1 |
| Batch 2 | 2.5 | 7.0 | 12.8 | 5.1 |
| Batch 3 | 2.1 | 6.7 | 12.7 | 6.0 |
| Batch 4 | 1.3 | 4.3 | 12.0 | 9.2 |

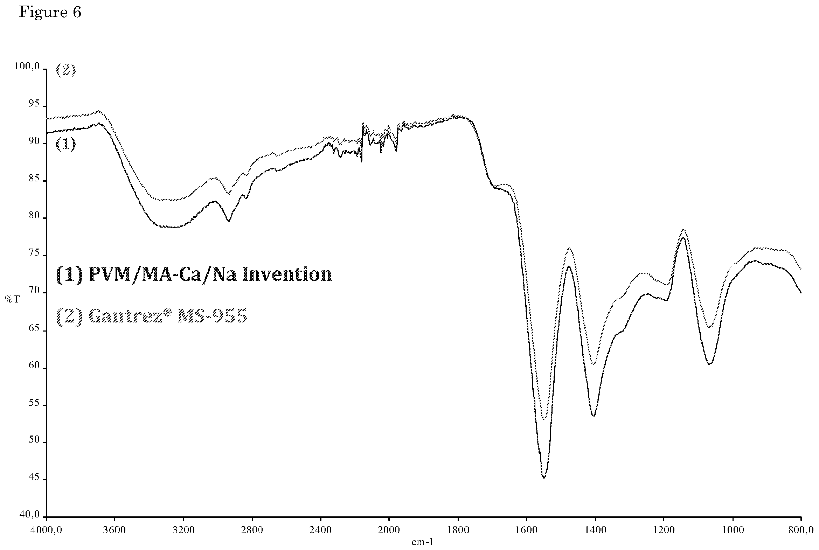

In order to rule out the possibility that the differences in adhesion profiles was not due to a difference in the chemical composition of the polymers, the two polymer powders were compared by FTIR analysis as shown in Figure 6. As can be seen in the FTIR spectrograms of the individual polymers, the individual spectrograms are almost identical which indicates that the two polymers are of similar chemical composition.

## Claims

1. A composition of powders of partial mixed metal salts of C₁-C₄ alkyl vinyl ether-maleic acid copolymers having:
(i) a median particle size by volume (Dv50) of less than or equal to 25 microns; a maximum particle diameter below which 90% of the sample volume exists (Dv90) of less than or equal to 50 microns; and
(ii) a particle uniformity reflected by a ratio between the maximum particle diameter below which 90% of the sample volume exists and the maximum particle diameter below which 10% of the sample volume exists (Dv90/Dv10 ratio) of less than or equal to 10, preferably wherein the Dv90/Dv10 ratio is less than or equal to 6;
wherein Dv10, Dv50 and Dv90 are measured according to the example in the description,
and wherein from about 10 to about 90 mol % of the carboxylic units in the polymer are converted to a mixture of metal salts.

2. The composition of claim 1, having a Dv50 of less than 15 microns, a Dv90 of less than 25 microns and a Dv90/Dv10 ratio of less than 6.

3. The composition of claim 1 or 2, in which the oversize fraction equal to or less than 1% by volume is not greater than 75 microns, preferably not greater than 50 microns.

4. The composition of any one of claims 1-3, wherein the metal salts are selected from the group consisting of sodium, calcium, strontium, zinc, magnesium, iron and potassium, preferably wherein the partial mixed salts are sodium and calcium salts, or calcium and zinc salts.

5. The composition of any one of claims 1-4 wherein said alkyl vinyl ether-maleic acid copolymers are of the general Formula I 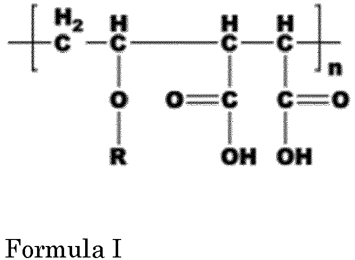 wherein R is C₁ to C₄ alkyl group, and the carboxylic acid functionality being a mixture of free acid and 10-90 mole % cationic salt function.

6. A personal care, oral care, health care or pharmaceutical product comprising the composition of powders of any one of claims 1-5.

7. An oral care product of claim 6, being a denture adhesive.

8. The denture adhesive of claim 7, comprising a partial calcium and sodium mixed salt of methyl vinyl ether-maleic acid copolymer, preferably a denture adhesive that contains 10-60 weight percent of the calcium and sodium mixed salt of methyl vinyl ether-maleic acid copolymer.

9. The denture adhesive of claim 8, comprising a partial calcium and zinc mixed salt of methyl vinyl ether-maleic acid copolymer.

10. A health care product of claim 6, being a wound dressing.

11. A health care product of claim 6, being a mucosal adhesive or bioadhesive.

12. A method of producing a composition of powders of any one of claims 1-5 comprising the steps of: (i) copolymerizing the C₁-C₄ alkyl vinyl ether with maleic anhydride in a suitable solvent; (ii) hydrolyzing the resultant maleic anhydride copolymer and reacting with metal cations either in the form of a base or a salt or an oxide in an aqueous medium to give the partial mixed metal salt; (iii) drying the mixed metal salt copolymer and (iv) milling, preferably jet-milling, the mixed metal salt copolymer to obtain particles having the characteristics as described in any one of claims 1-3.

13. The method of claim 12, wherein the copolymer is mixed with at least one co-ingredient prior to drying or milling and then milled together to give the final powder product, preferably wherein the co-ingredient(s) include one or more of the following: carboxymethylcellulose and its sodium salt, polyvinylpyrrolidone, alginates, polyacrylic acid based copolymers, polymethacrylate based copolymers, poly(ethylene oxide), silicon dioxide, natural gums, cellulose derivatives, saccharide derivatives, synthetic polymers and mixtures thereof.

## Patentansprüche

1. Zusammensetzung von Pulvern von teilweise gemischten Metallsalzen von C 1-C4-Alkylvinylether-Maleinsäure-Copolymeren mit:
(i) einer mittleren Teilchengröße nach Volumen (Dv50) von weniger als oder gleich 25 Mikrometern; einem maximalen Teilchendurchmesser, unter dem 90 % des Probenvolumens (Dv90) existiert, von weniger als oder gleich 50 Mikrometern; und
(ii) einer Teilchengleichförmigkeit, die durch ein Verhältnis zwischen dem maximalen Teilchendurchmesser, unter dem 90 % des Probenvolumens existiert, und dem maximalen Teilchendurchmesser, unter dem 10 % des Probenvolumens (Dv90/Dv10-Verhältnis) existiert, von weniger als oder gleich 10 widergespiegelt wird, wobei das Dv90/Dv10-Verhältnis vorzugsweise weniger als oder gleich 6 beträgt;
wobei Dv10, Dv50 und Dv90 gemäß dem Beispiel in der Beschreibung gemessen werden,
und wobei etwa 10 bis etwa 90 % der Carboxyleinheiten im Polymer in eine Mischung von Metallsalzen umgewandelt werden.

2. Zusammensetzung nach Anspruch 1 mit einem Dv50 von weniger als 15 Mikrometern, einem Dv90 von weniger als 25 Mikrometern und einem Dv90/Dv10-Verhältnis von weniger als 6.

3. Zusammensetzung nach Anspruch 1 oder 2, wobei die Überkornfraktion, die gleich oder weniger als 1 Vol.-% ist, nicht größer als 75 Mikrometer, vorzugsweise nicht größer als 50 Mikrometer ist.

4. Zusammensetzung nach einem der Ansprüche 1 bis 3, wobei die Metallsalze aus der Gruppe ausgewählt sind, die aus Natrium, Calcium, Strontium, Zink, Magnesium, Eisen und Kalium besteht, wobei die teilweise gemischten Salze vorzugsweise Natrium- und Calciumsalze oder Calcium- und Zinksalze sind.

5. Zusammensetzung nach einem der Ansprüche 1 bis 4, wobei die Alkylvinylether-Maleinsäure-Copolymere die allgemeine Formel I aufweisen 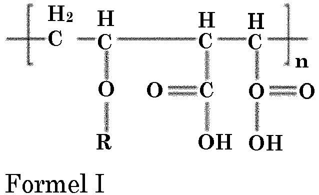 wobei R eine C₁ bis C₄-Alkylgruppe ist, und die Carbonsäurefunktionalität eine Mischung aus freier Säure und 10-90 Mol-% kationischer Salzfunktion ist.

6. Körperpflege-, Mundpflege-, Gesundheitspflege- oder pharmazeutisches Produkt, das die Zusammensetzung von Pulvern nach einem der Ansprüche 1-5 umfasst.

7. Mundpflegeprodukt nach Anspruch 6, das ein Gebisskleber ist.

8. Gebisskleber nach Anspruch 7, der ein teilweise gemischtes Calcium- und Natriumsalz von Methylvinylether-Maleinsäure-Copolymer, vorzugsweise einen Gebisskleber umfasst, der 10-60 Gew.-% des gemischten Calcium- und Natriumsalzes von Methylvinylether-Maleinsäure-Copolymer umfasst.

9. Gebisskleber nach Anspruch 8, der ein teilweise gemischtes Calcium- und Zinksalz eines Methylvinylether-Maleinsäure-Copolymers umfasst.

10. Gesundheitspflegeprodukt nach Anspruch 6, das ein Wundverband ist.

11. Gesundheitspflegeprodukt nach Anspruch 6, das ein Schleimhautkleber oder Bioadhäsiv ist.

12. Verfahren zur Herstellung einer Zusammensetzung von Pulvern nach einem der Ansprüche 1 bis 5, das die Schritte umfasst: (i) Copolymerisieren des C₁-C₄-Alkylvinylethers mit Maleinsäureanhydrid in einem geeigneten Lösungsmittel; (ii) Hydrolysieren des resultierenden Maleinsäureanhydrid-Copolymers und Umsetzen mit Metallkationen entweder in Form einer Base oder eines Salzes oder eines Oxids in einem wässrigen Medium, um das teilweise gemischte Metallsalz zu bilden; (iii) Trocknen des gemischten Metallsalz-Copolymers und (iv) Mahlen, vorzugsweise Strahlmahlen, des gemischten Metallsalz-Copolymers, um Teilchen mit den in einem der Ansprüche 1 bis 3 beschriebenen Eigenschaften zu erhalten.

13. Verfahren nach Anspruch 12, wobei das Copolymer vor dem Trocknen oder Mahlen mit wenigstens einem Co-Bestandteil gemischt und dann zusammen gemahlen wird, um das endgültige Pulverprodukt zu ergeben, wobei der/die Co-Bestandteil(e) vorzugsweise einschließen: Carboxymethylcellulose und ihr Natriumsalz, und/oder Polyvinylpyrrolidon, und/oder Alginate, und/oder Copolymere auf Polyacrylsäurebasis, und/oder Copolymere auf Polymethacrylatbasis, und/oder Poly(ethylenoxid), und/oder Siliciumdioxid, und/oder natürliche Gummis, und/oder Cellulosederivate, und/oder Saccharidderivate, und/oder synthetische Polymere und Mischungen davon.

## Revendications

1. Composition de poudres de sels de métaux mixtes partiels de copolymères d'alkylvinyléther en C₁-C₄-acide maléique présentant :
(i) une taille moyenne de particule en volume (Dv50) inférieure ou égale à 25 microns ; un diamètre maximal de particule en dessous duquel 90 % du volume d'échantillon existe (Dv90) inférieur ou égal à 50 microns ; et
(ii) une uniformité de particule reflétée par un rapport entre le diamètre maximal de particule en dessous duquel 90 % du volume d'échantillon existe et le diamètre maximal de particule en dessous duquel 10 % du volume d'échantillon existe (rapport Dv90/Dv10) inférieur ou égal à 10, de préférence dans laquelle le rapport Dv90/Dv10 est inférieur ou égal à 6 ;
dans laquelle Dv10, Dv50 et Dv90 sont mesurés selon l'exemple dans la description,
et dans laquelle d'environ 10 à environ 90 % en mole des unités carboxyliques dans le polymère sont convertis en un mélange de sels de métaux.

2. Composition selon la revendication 1, présentant une Dv50 inférieure à 15 microns, une Dv90 inférieure à 25 microns et un rapport Dv90/Dv10 inférieur à 6.

3. Composition selon la revendication 1 ou 2, dans laquelle la fraction surdimensionnée inférieure ou égale à 1 % en volume n'est pas supérieure à 75 microns, de préférence pas supérieure à 50 microns.

4. Composition selon l'une quelconque des revendications 1-3, dans laquelle les sels de métaux sont choisis dans le groupe consistant en sodium, calcium, strontium, zinc, magnésium, fer et potassium, de préférence dans laquelle les sels mixtes partiels sont des sels de sodium et calcium, ou des sels de calcium et zinc.

5. Composition selon l'une quelconque des revendications 1-4, dans laquelle lesdits copolymères d'alkylvinyléther-acide maléique sont de la formule générale I 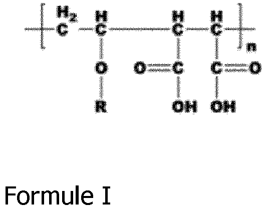 dans laquelle R est un groupe alkyle en C₁ à C₄, et la fonctionnalité acide carboxylique étant un mélange d'acide libre et de 10-90 % en mole de fonction de sel cationique.

6. Produit pour le soin de la personne, le soin oral, le soin de la santé ou pharmaceutique comprenant la composition de poudres selon l'une quelconque des revendications 1-5.

7. Produit pour le soin oral selon la revendication 6, étant un adhésif de prothèse dentaire.

8. Adhésif de prothèse dentaire selon la revendication 7, comprenant un sel mixte de calcium et sodium partiel de copolymère de méthylvinyléther-acide maléique, de préférence un adhésif de prothèse dentaire qui contient 10-60 pourcents en masse du sel mixte de calcium et sodium de copolymère de méthylvinyléther-acide maléique.

9. Adhésif de prothèse dentaire selon la revendication 8, comprenant un sel mixte de calcium et zinc partiel de copolymère de méthylvinyléther-acide maléique.

10. Produit pour la santé selon la revendication 6, étant un pansement pour plaies.

11. Produit pour la santé selon la revendication 6, étant un adhésif ou bioadhésif pour muqueuses.

12. Procédé de production d'une composition de poudres selon l'une quelconque des revendications 1-5 comprenant les étapes de : (i) copolymérisation de l'alkylvinyléther en C₁-C₄ avec de l'anhydride maléique dans un solvant approprié ; (ii) hydrolyse du copolymère d'anhydride maléique résultant et réaction avec des cations métalliques soit dans la forme d'une base soit d'un sel soit d'un oxyde dans un milieu aqueux pour fournir le sel de métal mixte partiel ; (iii) séchage du copolymère de sel de métal mixte et (iv) broyage, de préférence broyage par jet, du copolymère de sel de métal mixte pour obtenir des particules ayant les caractéristiques comme décrites dans l'une quelconque des revendications 1-3.

13. Procédé selon la revendication 12, dans lequel le copolymère est mélangé avec au moins un co-ingrédient avant séchage ou broyage et ensuite broyé conjointement pour fournir le produit de poudre final, de préférence dans lequel le(les) co-ingrédient(s) incluent un ou plusieurs des suivants : carboxyméthylcellulose et son sel de sodium, polyvinylpyrrolidone, alginates, copolymères à base de poly(acide acrylique), copolymères à base de polyméthacrylate, poly(oxyde d'éthylène), dioxyde de silicium, gommes naturelles, dérivés de cellulose, dérivés de saccharide, polymères synthétiques et mélanges de ceux-ci.
